# EUROPEAN PATENT APPLICATION

(11) **EP 2 292 223 A1**
(43) Date of publication of application: **09.03.2011**
(21) Application number: 10174886.1
(22) Date of filing: 15.10.1999
(51) Int. Cl.: A61K 31/20, A23L 1/30, A23K 1/16

(54) **Polyunsaturated fatty acid supplements**

(30) Priority: 15.10.1998 EP 98308403
(62) Divisional of application: 07001289.3
(71) Applicant: DSM IP Assets B.V., 6411 TE Heerlen (NL)
(72) Inventor: Streekstra, Hugo, 1017 SP, AMSTERDAM (NL); Waterschoot, Van, Isabel Antonia Maria, 8302 AB, EMMELOORD (NL)
(74) Representative: Elkenbracht, Johan Christiaan

(57) **Abstract**

Edible formulations, such as polyunsaturated fatty acids (PUFAs) such as pharmaceutical compositions or nutritional supplements, are disclosed comprising arachidonic acid (ARA). They are adapted to deliver from 150mg to 1g per day of ARA and may contain other PUFAs, for example docosahexaenoic acid (DHA). The DHA dosage is from 400 to 600mg per day, and the ratio of ARA:DHA may be from 1:5 to 5:1. Pharmaceutical compositions comprising ARA and DHA at a ratio of ARA:DHA of 1:1 to 1:2 are also disclosed, as are foodstuffs comprising of 0.1 to 5% ARA. Such formulations can be used to increase ARA levels *in vivo,* for example in pregnant women or for people who have diseases or conditions associated with low ARA levels.

## Description

This invention relates to the provision of polyunsaturated fatty acids (PUFAs) in the diet of humans and animals. More specifically it relates to the provision of polyunsaturated fatty acids of the n-6 and the n-3 families, and in particular the n-6 fatty acid arachidonic acid (ARA) and the n-3 fatty acid docosahexaenoic acid (DHA), and ratios thereof in balanced amounts.

The invention is in part based on the finding that an optimal balance of the n-6 and n-3 families can play a significant role in health and the prevention of chronic diseases. The main reason for this is that the two families compete for the same enzyme(s) for the formation of the long-chain members from their C18 precursors. As a consequence, and this occurs in prior art compositions, a surplus of member(s) of one family tends to depress the amount of the other family. Moreover, the members of the two families can in some circumstances have adverse effects on essential functions in the body, such as blood clotting and the immune response.

### Introduction

It is technologically relatively easy to provide the C18 n-6 fatty acid linoleic acid in the diet, since this fatty acid is abundantly present in common vegetable oils, such as corn oil and soy oil. There are also plant oils available that contain the C18 n-3 fatty acid α-linolenic acid, for instance rape seed oil, but these are much less readily used due to their lower stability. This usually leads to a surplus of the n-6 family over the n-3 family in the modern diet.

It has therefore been argued that n-3 fatty acids should be supplemented in many cases where a relative depletion is suspected. Generally this cannot be achieved by providing the C-18 precursor, since the efficiency of its conversion to C20 and C22 derivatives is low. Therefore, the consensus is that the C20 and C22 n-3 fatty acids (EPA and DHA) should be provided themselves.

In many cases the rationale behind this supplementation is to attenuate the action of the long-chain n-6 fatty acid ARA. It has been shown that the addition of the n-3 PUFAs, either derived from fish oil or from microbial (algae) oils does indeed lead to lower ARA levels. In the case of fish oil this occurs in spite of the fact that fish oil contains low amounts of ARA.

This depression of the ARA content is not always desirable. The invention thus seeks to provide preparations that may enhance the DHA and/or EPA status of animals, without adversely affecting ARA levels, or, conversely, enhance ARA without affecting the DHA and/or EPA status.

The use of preparations containing both ARA and n-3 PUFAs has been described before in the provision of PUFAs to infant formula. The rationale behind this is that human breast milk contains appreciable amounts of ARA and DHA which are considered useful to the developing infant.

In contrast, for adult nutrition there is no such natural source of PUFAs, although both ARA and DHA can be found as components of the human diet. However, for a number of reasons these PUFA levels appear to be sub-optimal. Furthermore, different populations have different levels of these PUFAs and this can affect the suitable dosage. As there is no model from nature, the relative amounts of PUFAs to be used needs to be determined and the present invention seeks to address this problem and provide various formulations and proportions of the PUFAs for certain applications.

### Prior Art

M. Makrides et al, European Journal of Chemical Nutrition 50:352-357 (1996) refers to a study to assess the effect of varying the internal intake of DHA (from 0 to 1.3g DHA/day) on breast milk fatty acids. DHA in the diet fed to lactating mothers had a strong specific and dose-dependent effect on breast milk DHA but did not affect ARA levels. This study used algae oils available from Martek Corporation, USA, under the brand name NEUROMINS™.

WO-A-92/12711 (Martek) refers to oil blends containing ARA and DHA, for example an ARA:DHA ratio of 3:1 to 2:1, in particular to provide levels of these PUFAs in infant formula in amounts comparable to human breast milk (which has an ARA level of 0.5 to 0.6%).

A number of PUFA-containing compositions are currently marketed. EFANATAL™ are capsules, two capsules to be taken per day to give a daily intake of DHA (125mg), ARA (8.6mg) and GLA (40mg). The capsules contain an oil that is primarily based on fish oil. The Applicant has found that this decreases *in vivo* ARA levels, because the DHA content relative to the ARA content in the capsules is too high. Thus this product is in fact an ARA lowering, rather than ARA increasing, composition despite the fact that it contains ARA. A comparison between this product and those of the invention is provided later.

EFAMARINE™ is also capsules, containing primarily fish and evening primrose oils, of which two are to be taken per day to give a daily intake of EPA (34mg), DHA (22mg) and GLA (68mg).

EFALEX™ is an oil blend, where a teaspoon (5ml) is intended to be taken twice a day, each teaspoon giving DHA (100mg), GLA (21mg), ARA (8mg) and thyme oil (6mg).

### Summary of the Invention

A first aspect of the present invention relates to an edible formulation comprising ARA in an amount adapted to deliver a dosage (of ARA) of from 150mg to 1g per day.

Preferably the formulation is adapted to deliver from 200 to 900mg per day ARA, such as from 200 to 700mg per day, optimally from 250 to 400 or 500mg per day.

Edible formulations include dietary supplements and (pharmaceutical) formulations and preparations, such as tablets, pills and capsules. They additionally include (solid or liquid) foodstuffs, for example dairy products (margarine, butter, milk, yoghurt), bread, cakes; drinks such as beverages (tea, coffee, cocoa, chocolate drinks), fruit juices, soft (e.g. fizzy) drinks; confectionery; oily foods (snacks, salad dressing, mayonnaise), soups, sauces, carbohydrate-rich foods (rice, noodles, pasta), fish-containing foods, baby foods (such as infant formula, either as a liquid or powder), pet food, and ready prepared or microwaveable foods.

The ARA can be from any suitable source. It may be from a natural (e.g. vegetable or marine) source, or it may be from a microbial source or from a microorganism, such as fungus, bacterium or a yeast.

Suitable fungi are of the order *Mucorales,* for example *Mortierella, Pythium* or *Entomophthora.* The preferred source of ARA is from *Mortierella alpina or Pythium insidiosum.* Suitable commercially available ARA oils include those from DSM/Gist-brocades, Wateringseweg, P.O. Box 1, 2600 MA, Delft, The Netherlands under the trade mark OPTIMAR™ and from Martek Corporation, 6480 Dobbin Road, Columbia, MD 21045, USA, under the trade mark ARASCO™.

In addition to the ARA, one or more additional PUFAs may be provided. This may be another n-6 PUFA in addition to ARA (such as a C18, C20 or C22 fatty acid) or it may be a n-3 fatty acid (for example, a C18, C20 or C22 fatty acid) and in particular EPA and/or DHA. Each PUFA that may be used in the invention may be in the form of a free fatty acid, fatty acid ester (e.g. methyl or ethyl ester) as a phospholipid or as a triglyceride.

If the formulation comprises an n-3 fatty acid, it is preferred that this is EPA or DHA. If it is DHA, then the formulation is preferably adapted to deliver the same dosage as specified for ARA, such as from 400 to 600mg per day DHA. Alternatively, or in addition, if the formulation comprises EPA, then it is preferably adapted to deliver a dosage of from 150mg to 1g per day EPA, such as from 250 to 500mg of EPA per day.

If the formulation is to be taken (eaten or ingested) once a day then it can contain from 150mg to 1g of ARA. If twice a day then the formulation can have 75mg to 0.5g of ARA, for three times a day a content of 50mg to 330g ARA, and so on, pro rata, for more frequent administrations. The same calculations can be applicable for other PUFAs that may be present, such as DHA.

If the formulation comprises more than one PUFA then the amount of each PUFA can be expressed relatively, as a ratio. For example, if an n-3 PUFA is additionally provided, then the ratio of ARA:n-3 PUFA (such as DHA or EPA) can be from 1:5 to 5:1, preferably from 2:1 to 1:3, optimally from 1:1 to 1:2. The relative amounts of the PUFAs can be balanced so that PUFA levels are supplemented, increased (or at least not decreased significantly) bearing in mind the condition of the individual.

Preferably the PUFA is present in an oil. This may be a pure oil, a processed (e.g. chemically and/or enzymatically treated) or concentrated oil. This oil may comprise from 10 to 100% of the PUFA, but the content may be from 20 to 45%, optimally from 30 to 45% of the desired PUFA, for example ARA, if a microbial oil. Of course, this oil may contain one or more PUFAs within these percentage concentrations. The oil may be a single oil derived from a single cell or a microbial source, or it may be a blend or mixture of two or more oils from these or other (e.g. vegetable or marine) sources. The oil may contain one or more antioxidants (e.g. tocopherol, vitamin E, palmitate) for example at a concentration of from 50 to 800ppm, such as 100 to 700ppm. Suitable processes for preparing PUFAs are described in International patent application numbers PCT/EP97/01446 (WO-A-97/36996), PCT/EP97/01448 (WO-A-97/37032), and PCT/US92/00517 (WO-A-92/13086).

A second aspect of the invention relates to a (pharmaceutical) composition comprising ARA and DHA at a ratio of ARA:DHA of from 1:1 to 1:2. This ratio of PUFAs has been found to provide a good balance, and can increase in vivo DHA levels without ARA levels being suppressed due to a too high DHA content. The DHA can be from a natural (e.g. marine) source or from a microbial source (e.g. from an algae).

A third aspect relates to an edible formulation (eg. a foodstuff) comprising from 0.1 to 3 or 5% ARA. Preferably, the amount is from 0.5 to 1.5 or 2%, optimally from 0.3 to 0.8%. Suitable foodstuffs have already been discussed in relation to the first aspect. Preferred methods of preparing infant formula are disclosed in International application numbers PCT/EP97/01447 (WO-A-97/35487) and PCT/EP97/01449 (WO-A-97/35488).

Suitable formulations can include oils, for example to be taken orally. The oil may be taken as such, or it may be encapsulated, for example in a shell, and may thus be in the form of capsules. The shell or capsules may comprise gelatin and/or glycerol. The formulation may contain other ingredients, for example flavourings (e.g. lemon or lime flavour).

The invention has found use in improving PUFA levels in normal, healthy, well fed individuals (who would normally not be expected to benefit if on an adequate diet). However it can also be used with individuals with low PUFA level(s) or deficiencies.

Thus, a fourth aspect of the present invention relates to the use of ARA (eg. as a dietary or nutritional supplement or for the manufacture of a medicament) for a woman who is:
a. pregnant and at an age of from 15 to 20;
b. pregnant and at an age of from 40 to 60, such as from 50 to 55;
c. pregnant with her fourth, fifth or subsequent child;
d. pregnant with twins, triplets or quadruplets;
e. pregnant and is from 1 to 3 months into her pregnancy;
f. pregnant as a result of *in vitro* fertilisation (IVF) or who is undergoing IVF treatment (which includes enrolling in or participating in an IVF procedure) but not yet pregnant;
g. pregnant at from 20 or more weeks of gestation;
h. pregnant and is malnourished, poorly or marginally nourished, suffering from malnutrition or malabsorption or deficient in one or more essential fatty acids (such as a PUFA);
i. trying to become pregnant;
j. pregnant, for promoting the intra-uterine growth or health of a foetus; or
k. lactating, for increasing the level of ARA or EPA in the woman's breast milk.

In the case of (h) these conditions are relatively rare in Western Europe, but may be found in women in Africa or some Asian countries (eg. Pakistan).

For pregnant women, the benefit to the foetus in (j) has not always been predictable or immediately apparent due to the variance in individuals in the transport of fluid between the mother and foetus. The placenta to foetus connection (the umbilical cord) can vary in size and physiological condition and so in the past the supplementation of the mother with PUFAs has not necessarily indicated that the foetus will receive these PUFAs and so benefit also.

A fifth aspect relates to the use of ARA (as a dietary or nutritional supplement) for a human (male or female) over 50 years old, preferably over 65 years old.

A sixth aspect relates to the use of ARA (as a dietary or nutritional supplement) for a non-human mammal which is pregnant or lactating.

The ARA is preferably ingested at from 150 to 700mg per day, optimally from 250 to 500mg per day.

A seventh aspect of the present invention relates to the use of ARA for the manufacture of a medicament for (assisting in) the prophylaxis, prevention, amelioration or treatment of a disease or condition associated with an abnormal or low level of an n-3 or n-6 PUFA, for example in the blood. The invention therefore finds use in subjects that have low levels of ARA, for example for those that cannot or cannot effectively convert linoleic acid (LA) to ARA. Therefore, suitable patients may have a malfunctioning, inefficient or deficiency in Δ6-desaturase.

A (mouse) model of PUFA deficiency has been established and used to mimic the effects of malnourishment. This model has shown the beneficial effects of the formulations of the invention, including during pregnancy, for both the mother and foetus. It has also allowed simulation of poor placental transfer and intra-uterine growth retardation, and shown the benefits of supplementation with formulations of the invention in the individuals mentioned in the various aspects of the invention (and the foetus if pregnant).

The Applicant has found that certain diseases or conditions, in particular neuronal diseases, are associated with low levels of *in vivo* PUFAs, in particular low levels of ARA in the blood. It is therefore thought that the administration of ARA, or a balance of the PUFAs, will be able to assist in the prophylaxis, prevention, amelioration or treatment of these diseases or conditions. The diseases in question include: neuronal disease, such as schizophrenia, cystic fibrosis, idiopathic immunoglobulin A nephropathy, multiple sclerosis, retinitis pigmentosis, Usher's syndrome, celiac disease, macular degeneration, Parkinsons' disease, osteoporosis, Alzheimer's disease or phenylketonuria.

An eighth aspect relates to the use of ARA, optionally with DHA, for promoting lactation and/or reproductive efficiency or success or fertility in a human or non-human female mammal.

A ninth aspect of the present invention relates to the use of ARA and DHA (in an edible formulation) at an ARA:DHA ratio that increases the ARA level in blood. Preferably the ratio of ARA:DHA is from 1:5 to 5:1, such as from 1:1 to 1:2.

The invention is particularly application to those people that have low ARA levels, for example a diabetic, alcoholic, drug abuser, smoker or a subject having an abnormal or low immune level or who is immunocompromised.

The use of the fourth to ninth aspects include methods of administration of the ARA (and optionally DHA), either as such or in a formulation, to a subject (individual, human or animal) where that subject is in need of, or will benefit from, the administration, or those uses in the manufacture of a medicament for the purposes specified. Formulations may exclude GLA and/or DGLA if necessary.

The dose or amount of ARA (and DHA, if present) is preferably such that it increases either an essential fatty acid (EFA) sufficiency index (defined as the level of 20:4 n-6 (ARA) divided by the level of 20:3 n-9 fatty acid (mead acid)) and/or an EFA balance index (defined as the level of 22:6 n-3 (DHA) divided by the level of 22:5 n-6). Here, levels include those in the blood (eg. in red blood cells), brain, placenta, liver, intestine, plasma or foetus.

Preferred features and characteristics of one aspect of the invention are equally applicable to another aspect *mutatis mutandis.*

The following Examples are provided to merely illustrate the invention, and are not to be construed to be limiting.

### Examples 1 to 3: Preparation of a composition containing balanced proportions of PUFAs.

This example describes the blending of n-6 and n-3 oils so that they can be included in a single capsule.

The composition was prepared by combining one n-6 PUFA-rich oil with three different n-3 PUFA-rich oils. The n-6 PUFA-rich oil was derived from the fermentation of the filamentous fungus *Mortierella alpina,* and contained approximately 40% ARA as the major fatty acid. For the n-3 PUFA-rich oil the three different sources were: a high-EPA (above 45%) low-DHA (about 10%) fish oil (from Pronova, Norway under the trade name EPAX™, product no. EPAx4510TG), a high-DHA (above 50%) low-EPA (about 20%) fish oil (also from Pronova under the same brand name, product no. EPAx2050TG), and an oil derived from fermentation of the unicellular alga *Crypthecodinium cohnii* which contains 40% DHA as major fatty acid but is virtually devoid of EPA (from Martek Corporation, Columbia, United States of America under the trade name DHASCO™).

The oils were mixed in appropriate quantities to give the desired amounts and proportions of n-3 and n-6 PUFAs. Here the ARA:DHA ratio for the three blends (Examples 1 to 3) was 1:1. During this procedure, the oxidation-sensitive oils were protected from environmental oxygen by a blanket of oxygen-free nitrogen gas. Subsequently, the oils were used to prepare soft-gel gelatin capsules, where each capsule had 400mg ARA and 400mg DHA.

### Example 4: Provision of balanced PUFAs to pregnant women during the early or latter stages of pregnancy.

This Example concerns the trial of pregnant women that are supplemented with ARA and DHA either between weeks 6 and 15 or between weeks 20 and 25 during pregnancy until delivery (birth). The ARA source was a triglyceride oil containing 38% ARA available from DSM/Gist-brocades, Delft, The Netherlands, under the trade name OPTIMAR™. This is an oil produced by the fungus *Mortierella alpina.* For DHA either a DHA-rich fish oil of food grade or an algae-derived oil obtained from Martek Corporation under the trade mark DHASCO™ was employed.

Maternal supplementation of ARA and DHA during pregnancy was therefore studied to see if the fatty acid status of the mother measured at birth and subsequently during lactation compared with the controlled group that received no supplementation. The measurements included maternal erythrocyte ARA and DHA values, ARA and DHA content of the umbilical arteries and venous vessel wall, ARA and DHA content of breast milk.

The study was a case controlled study involving 10 pregnant women. One experimental group (of five women) received one or more gelatin capsule (each of 250mg ARA) oil per day (containing 38% ARA) and one capsule (each of 500mg DHA) oil per day (containing 25% DHA). The control group received the same amount of placebo gelatin capsules to overcome differences in daily calorie intake. The vitamin E intake of the experimental and controlled groups was equal, and the capsules were taken during breakfast.

Blood samples were taken at the beginning of the trial and at the end of gestation. Red blood cell fatty acids were measured (as phospholipids) using capillary gas chromatography with flame ionisation.

It was found that the supplemented women had significantly higher levels of both DHA and ARA in the red blood cells during pregnancy and at the time of birth. Remarkably, these higher levels persisted during the lactation period, being apparent both in the red blood cells of the mothers and their breast milk. The ARA level in breast milk was found to have risen to from 0.8 to 1.0% ARA. In addition the ARA levels in the blood of the newly born children was found to be higher than the control group. This finding is of major significance for mothers and their children under marginal nutritional conditions.

### Example 5: Provision of balanced PUFAs to elderly people.

The Applicant perceives a need to enhance the n-3 PUFA status of the population, not in the least in the elderly population, where diseases such as Parkinson's disease and Alzheimer's disease have been found to be associated with a low PUFA status. This is thought to be partly due to inefficient or deficient Δ6-desaturase enzyme. However care is needed, especially in older people, since a decrease in ARA levels could exert a negative effect on the immune system.

A formulation was prepared according to Example 1, containing n-3 and n-6 PUFAs in a ratio of DHA:ARA of 2:1. The capsules were given to a group of healthy, elderly men and women (at least 65 years of age), at a dosage of 1 g n-3 PUFAs per day.

After one month the PUFA status of the red blood cells of the subjects was assessed. It was found that in all cases the levels of DHA had increased, whereas the levels of ARA had remained constant, or showed a slight increase in some cases. Thus it was possible to enhance the n-3 PUFA status of patients, without compromising the ARA status, by the use of a balanced formulation.

### Example 6: Provision of PUFAs to pregnant women.

Two types of PUFA-containing capsules were prepared. The first contained ARA, at 500mg per capsule. These were to be taken one a day. The ARA was provided as a microbial oil, obtained from DSM/Gist-brocades, Delft, The Netherlands, under the trade name OPTIMAR™. These capsules had a gelatin coat, and contained 20mg of vitamin E. Similar capsules were also prepared having the same amount (500mg) of DHA, being present as a microbial oil obtained from Martek Corporation, Columbia, United States of America (under the trade name DHASCO™) These capsules were also designed to be taken one per day.

Trials were conducted with pregnant women ingesting either one ARA capsule per day, or one ARA and one DHA capsule per day. The women chosen for the study were those that had been found to have relatively low levels of ARA in the blood. A number of women who were pregnant were therefore tested for *in vivo* ARA blood levels and permission was obtained to take part in the study. The first group of women were teenagers of from 15 to 20 years of age. For all these women, this was their first pregnancy. Due to early maturation they were found to benefit from both ARA and ARA plus DHA supplementation in their diet. Both regimes increased in vivo ARA levels.

A second group of women, also pregnant, were studied, these being from age 40 to 50. During pregnancy it was also found their in vivo blood levels were increasing under both supplementation regimes. Half of the women chosen in this study were having their fourth child.

Three women each pregnant with twins were chosen for supplementation with one ARA capsule and one DHA capsule per day. Their ARA *in vivo* levels were found to be relatively low, probably because the ARA from the blood of the mother was being absorbed and consumed by both foetuses. These women were supplemented with the ARA and DHA capsules and the ARA levels in the blood were found to increase.

### Example 7: Provision of ARA and DHA to subjects with low PUFA content.

The same capsules were used as described in Example 6, except this time the ARA capsules contained only 250mg ARA. These capsules could be taken once or twice daily, according to the subject and their condition.

A number of people were chosen for this study due to their relatively low content of PUFAs in the blood. The reason for the low PUFA content was not always immediately evident. However, it has been found that a number of diseases or adverse conditions lead to low PUFA levels, and it was therefore postulated that providing either a correct dosage of ARA, or a balance of ARA:DHA, the in vivo ARA levels could be increased, which might moderate some of the symptoms of the condition. Some of the conditions were thought to result in a poor efficiency in conversion of a precursor to ARA itself, for example a defect or deficiency with the enzyme Δ6-desaturase. Those conditions that were found by the Applicant to often give rise to low PUFA levels included cystic fibrosis, multiple sclerosis, celiac disease and osteoporosis. In addition, patients who were being treated for alcoholism, addiction to drugs or who were immunocompromised (AIDs patients) were also found to have low levels of PUFAs.

A study was therefore made where either one or two ARA capsules were taken daily, to give an ARA:DHA content of either 1:1 or 1:2. In almost all cases those subjects who were taking these capsules (for at least 3 weeks) were all found to have, at the end of the trial, increased *in vivo* ARA blood levels.

### Example 8: Provision of PUFAs in infant formula.

Both solid (powdered) and liquid infant formula baby food was prepared containing 0.5% ARA and 0.5% DHA. This formula was fed to babies regularly in their first three months by mothers who had decided not to breast feed their children. As a control, the *in vivo* ARA blood levels of these children were compared to those that were being breast fed over the same time period. It was found that in the infants being bottle fed that their ARA levels were comparable to those being breast fed.

### Comparative Example 9 and Example 10

A number of breast feeding women were chosen for a comparative trial. One group of women were fed two EFANATAL™ capsules per day (to give a daily intake of DHA 125mg, ARA 8.6mg and GLA 40mg). For comparison, a second group of women were given similarly prepared capsules (with a gelatin/glycerol shell) containing 150mg ARA per capsules (to give a daily ARA intake of 300mg ARA,2 capsules per day). In this second group a third capsule was also taken, one per day, which contained DHA at 500mg per capsule.

The ARA levels in the lactating women in both groups, after child birth, was compared. Also compared was the level of ARA in the mothers breast milk.

In the first EFANATAL™ group the ARA levels were found to have decreased markedly in the blood, and to a lesser extent in the breast milk, only two weeks after the trial involving consumption of EFANATAL™ had begun. In contrast those women taking the two capsules of ARA and one capsule of DHA per day were found to have the ARA levels in their blood increase, and the breast milk levels also increased to above 0.7%.

### Example 11: Amelioration of fatty acid deficiency in mouse pregnancy through supplementation with ARA and DHA.

A major problem during the pregnancy of humans and non-human mammals is the occurrence of intra-uterine growth retardation. This condition is associated with significant health risks for the infant after birth that may continue into adult life. The condition can develop even during pregnancy of an apparently healthy woman and is difficult to predict. It is generally assumed that it is caused by poor functioning of the placental interchange, for instance because the placenta is too small or in poor physiological condition.

This unpredictability has obstructed the development of a reliable animal model for this condition. In principle one could simulate a poor placental function by decreasing the blood flow through the umbilical vein, for instance by restricting its diameter by a clamp. The problem with this method is that it requires surgery of the pregnant animal, which can adversely affect both the foetus and the mother, and it is difficult to achieve a uniform decrease of the blood flow in this way. Therefore a different model has been developed. A poor placental function translates into a decreased supply of essential fatty acids (EFAs) to the foetus. In the 'natural' condition this is caused by a decreased blood flow, at an otherwise normal physiological concentration in the blood of the healthy mother. In the present example we have simulated this condition by decreasing the concentration of the essential fatty acids in the blood of the mother, but having a normal flow through the placenta. For this purpose an early phase of fatty acid deficiency in pregnant mice was induced. In this phase the deficiency was expressed in biochemical parameters, but functional defects were not apparent. Thus it was ensured that while the pregnancy proceeded in the normal way the supply of essential fatty acids to the foetus was restricted.

In the trial 40 female mice, 8-10 weeks of age, were fed a regular mouse chow diet for 1 week. Subsequently they were divided into 8 experimental groups: RID 1 to 4 and EFAD 1 to 4. The RD groups continued to receive a regular chow diet, containing 6.5% of fat. The EFAD groups received an essential fatty acid deficient diet. The numbers 1 to 4 indicate various lipid supplements, according to Table 1. ARA was from DSM, Delft, and DHA from Pronova (fish oil) as described in previous Examples.

**Table 1: Amounts of lipid supplements as percentage of total dietary lipids. The diets contained between 3.8% of 5.6% (g/g) lipids.**

| RD or EFAD | MCT (Medium-Chain Triglycerides) | ARA (Arachidonic Acid Oil) | DHA (Docosahexaenoic Acid Oil) |
|---|---|---|---|
| 1 | 19 | 0 | 0 |
| 2 | 15 | 4 | 0 |
| 3 | 4 | 0 | 15 |
| 4 | 0 | 4 | 15 |

The fatty acid composition of the RD (regular diet) and the EFAD (essential fatty acid deficient) diets as well as the oil supplements are given in Table 2.

**Table 2: Fatty acid composition of lipid fractions, expressed as g% of total fatty acids.**

| Fatty Acid | RD lipid | EFAD lipid | MCT | ARA oil | DHA oil |
|---|---|---|---|---|---|
| 8:0-12:0 | | | 100.00 | | |
| 14:0 | 0.10 | | | 1.90 | 3.60 |
| 16:0 | 10.00 | 44.78 | | 16.14 | 19.50 |
| 17:0 | 0.10 | | | | |
| 18:0 | 4.00 | 54.73 | | 12.10 | 5.11 |
| 20:0 | 0.30 | | | 0.85 | 0.34 |
| 22:0 | 0.30 | | | 1.48 | 0.29 |
| 24:0 | 0.20 | | | 1.55 | 0.18 |
| 18:3ω3 | 7.50 | | | | 0.58 |
| 18:4ω3 | | | | | 0.96 |
| 20:4ω3 | | | | | 0.39 |
| 20:5ω3 | | | | | 6.52 |
| 22:5ω3 | | | | | 1.33 |
| 22:6ω3(DHA) | | | | | 25.08 |
| 18:2ω6 | 55.00 | | | 7.01 | 1.74 |
| 18:3ω6 | | | | 3.24 | 0.20 |
| 20:2ω6 | | | | 0.38 | 0.30 |
| 20:3ω6 | | | | 3.85 | 0.11 |
| 20:4ω6 (ARA) | | | | 37.64 | 2.15 |
| 22:4ω6 | | | | | 0.41 |
| 22:5ω6 | | | | | 8.32 |
| 16:1ω7 | | | | | 6.00 |
| 18:1ω7 | | | | 0.45 | 2.77 |
| 18:1ω9 | 22.50 | 0.50 | | 13.01 | 12.60 |
| 20:1ω9 | | | | 0.36 | 0.96 |
| 22:1ω9 | | | | | 0.12 |
| 20:3ω9 | | | | 0.04 | |
| 24:1ω9 | | | | | 0.46 |

Two additional control groups were included. One group (RD 0) did not receive any lipid supplement. The second group received the same diet as RD 0, but served as a non-pregnant (NP) outgroup. The animals had unrestricted access to the diets.

The experimental groups were treated according to the time schedule shown below.

**Table 3: Time schedule of treatments.**

| Day | Treatment |
|---|---|
| day - 3 | Intraperitoneal injection of 5 IU Folligonan (FSH) IP (all groups except NP). Regular diet replaced by experimental diets |
| day - 1 | Intraperitoneal injection of 5 IU Chorulon (hHCG) IP (all groups except NP). Male mice introduced into the cages (all groups except NP) |
| day 0 | Males removed |
| day 15 | Animals killed by heart puncture under halothane anaesthesia (4-6% in N₂O/O₂ |

The hormone treatment with Folligonan™ and Chorulon™ (from Organon, the Netherlands) induced super-ovulation in the females. This procedure, combined with the short exposure to the males, gave a reasonable probability of pregnancy, but no guarantee. The fatty acid composition of various tissues or sections of both the pregnant mice and their foetuses was determined by gas chromatography. The fractionation, homogenisation and extraction of the various tissues was performed by methods known in the art.

On average, the animals consumed 3.9g of the diets per day, without significant differences between the various RD and EFAD groups. The dietary dosage of PUFAs is shown in Table 4.

**Table 4: Dietary dosage of ARA and DHA, expressed as a percentage of the lipid fraction and as mg intake per day.**

| | | ARA | | DHA | |
|---|---|---|---|---|---|
| No. | Diet | % of lipid | mg/day | % of lipid | mg/day |
| 0 | RD | 0 | 0 | 0 | 0 |
| 1 | RD+MCT | 0 | 0 | 0 | 0 |
| 2 | RD + ARA/MCT | 1.29 | 2.7 | 0 | 0 |
| 3 | RD+DHA/MCT | 0.30 | 0.5 | 3.30 | 5.1 |
| 4 | RD+ARA/DHA | 1.63 | 2.5 | 3.25 | 5.0 |
| 1 | EFAD+MCT | 0 | 0 | 0 | 0 |
| 2 | EFAD + ARA/MCT | 1.11 | 2.4 | 0 | 0 |
| 3 | EFAD+DHA/MCT | 0.34 | 0.5 | 3.73 | 5.9 |
| 4 | EFAD+ARA/DHA | 1.58 | 2.3 | 3.27 | 4.8 |

First it was checked whether the EFAD indeed induced a biochemically relevant essential fatty acid deficiency in the blood of the female mice. There were few differences in the blood levels of various fatty acids between pregnant and non-pregnant mice of the same dietary group as seen in the comparison with RD0 and NP (data not shown). Therefore these two groups were compared, to increase the statistical power of the comparison, except in the cases where there was a significant difference between pregnant and non-pregnant animals. In those cases, the values for the pregnant individuals was used. The results are shown in Table 5.

**Table 5: Levels of essential fatty acids (EFAs) in red blood cells of female mice.**

| PUFA (ratio) | RD+MCT | EFAD+MCT |
|---|---|---|
| 18:3 n-3 | 0.19 ± 0.02 | 0.05 ± 0.01 |
| 20:5 n-3 | 0.24 ± 0.02 | 0.09 ± 0.03 |
| 22:6 n-3 (DHA) | 6.38 ± 0.25 | 4.53 ± 0.28 |
| 18:2 n-6 | 8.43 ± 0.08 | 3.00 ± 0.12 |
| 20:4 n-6 (ARA) | 17.23 ± 0.44 | 18.87 ± 0.85 |
| EFA sufficiency index: 20:4 n-6/20:3 n-9 | 63 | 11 |
| EFA balance index: 22:6 n-3/ 22:5 n-6 | 11 | 4 |

The EFAD caused a marked decrease in the level of essential fatty acids, with the exception of arachidonic acid. However, in spite of the maintenance of the level of arachidonic acid, there was a marked (n-6) essential fatty acid deficiency. This is clearly seen in the EFA sufficiency index, the ratio between the level of arachidonic acid (20:4 n-6) and its non-essential analogue mead acid (20:3 n-9). This latter fatty acid accumulates only if there are insufficient essential fatty acids as substrates for normal biosynthesis: in that case the non-essential fatty acid oleic acid (18:1 n-9) is elongated and desaturated instead, leading to the formation of n-9 analogues of the physiological PUFAs. It is clear from Table 5 that this EFA sufficiency index dropped dramatically in the EFAD-fed mice.

Another index indicates the correct balance of n-3 and n-6 essential fatty acids. This EFA balance index is the ratio between DHA (22:6 n-3) and arachidonic acid (22:4 n-6). This index also strongly decreased in the EFAD group.

So these data shown that the EFAD diet indeed induced a clear biochemical EFA deficiency, as was intended.

It was then checked whether the addition of arachidonic acid and/or DHA to the diet would lead to alleviation of this deficiency in the red blood cells of the female mice. First the control data of the fatty acid sufficient (RD) mice are presented in Table 6.

**Table 6: Effect of PUFA supplementation on essential fatty acids in red blood cells of fatty acid-sufficient female mice. Fatty acid data expressed as percentage of the RD-group.**

| | RD | RD + ARA/MCT | RD + DHA/MCT | RD + AR.A/DHA |
|---|---|---|---|---|
| 18:3 n-3 | 100% | 93% | 74% | 82% |
| 22:6 n-3 (DHA) | 100% | 85% | 131% | 132% |
| 18:2 n-6 | 100% | 84% | 94% | 100% |
| 20:4 n-6 (ARA) | 100% | 107% | 79% | 93% |
| 20:4 n-6/20:3 n-9 | 63 | 59 | 59 | 67 |
| 22:6 n-3/22:5 n-6 | 11 | 9 | 15 | 14 |

It was found that addition of the supplements with either ARA or DHA depressed the levels of the other PUFA. In contrast, the combined supplement allowed the enhancement of the PUFA status, even in fatty acid sufficient mice. The supplement used caused a slight depression of the ARA status, causing an increase of the EFA balance index. This could be due to the ratio chosen, with DHA:ARA approximately at 2:1. Surprisingly, the EFA-sufficiency index was also enhanced by the supplement, even though these mice were apparently not fatty acid deficient.

It was then investigated whether the supplementation with PUFAs led to an improvement in the essential fatty acid status in the blood cells of the EFAD-fed animals.

**Table 7: Effect of PUFA supplementation on essential fatty acids in red blood cells of fatty acid-deficient female mice. Fatty acid data are expressed as a percentage of the RD-group.**

| | EFAD | EFAD + ARA/MCT | EFAD + DHA/MCT | EFAD + ARA/DHA |
|---|---|---|---|---|
| 18:3 n-3 | 24% | 37% | 25% | 34% |
| 22:6 n-3 (DHA) | 71% | 70% | 174% | 171% |
| 18:2 n-6 | 36% | 42% | 49% | 44% |
| 20:4 n-6 (ARA) | 109% | 121% | 70% | 90% |
| 20:4 n-6/20:3 n-9 | 11 | 43 | 43 | 67 |
| 22:6 n-3/22:5 n-6 | 4 | 5 | 16 | 16 |

Table 7 shows that the EFAD-mice responded quite strongly to the PUFA-supplement, especially in their DHA status. While there are no indications that supplementation with ARA depresses the DHA status, the converse is clearly true: the addition of the DHA supplement caused a clear depression of the ARA status. It is also clear that the addition of PUFAs specifically restored PUFA levels, with the levels of the C-18 fatty acids being much less affected. Interestingly, the combined supplement was the only one that caused full restoration of the EFA sufficiency index.

Finally it was investigated whether the enhancement of the PUFA status in the blood of the mother would lead to an improved status of the fetus. To this end we chose the head of the foetus as the most relevant compartment: the growth of the brain (and other neural tissue) is quantitatively the most important process depending on the provision of PUFAs.

The data for the foetuses of the RD-fed mothers are shown in Table 8.

**Table 8: Effect of PUFA supplementation of EFA-sufficient mothers on essential fatty acids in mice foetus heads. Fatty acid data in the RD-group is expressed as mol-percent. Fatty acid data for the experimental groups is expressed as percentage of the RD-group.**

| | RD | RD+ARA/MCT | RD+DHA/MCT | RD+ARA/DHA |
|---|---|---|---|---|
| 22:6 n-3 (DHA | 5.89 | 101% | 135% | 125% |
| 20:4 n-6 (ARA) | 11.87 | 103% | 93% | 102% |
| 20:4 n-6/20:3 n-9 | 27 | 30 | 28 | 40 |
| 22:6 n-3/22:5 n-6 | 8 | 6 | 18 | 13 |

The data show that the supplements caused modest changes in the concentrations of PUFAs in the heads of foetuses of the RD-fed mice. Surprisingly, there was a marked improvement in the EFA sufficiency index for the combined supplement, as opposed to the separate supplements. In addition, both DHA-containing supplements caused a significant increase in the EFA balance index.

**Table 9: Effect of PUFA supplementation of EFA-deficient mothers on essential fatty acids in mouse foetus heads. Fatty acid data expressed as percentage of the RD-group. The EFAD + AA/MCT group did not contain pregnant females.**

| | EFAD | EFAD+ARA/MCT | EFAD + DHA/ MCT | EFAD + ARA/ DHA |
|---|---|---|---|---|
| 22:6 n-3 (DHA) | 61% | - | 160% | 146% |
| 20:4 n-6 (ARA) | 98% | - | 76% | 83% |
| 20:4 n-6/20:3 n-9 | 9 | - | 14 | 17 |
| 20:6 n-3/22:5 n-6 | 2 | - | 33 | 24 |

The fatty acid deficiency of the foetuses was even more severe than that of the mothers. The PUFA-supplements led to a marked improvement of the EFA sufficiency index, almost restored to the RD-level. This was probably due to the relatively low dosage of arachidonic acid in the supplement, since the EFA balance index is even higher than in the foetuses of the RD-fed mothers. This implies that the PUFAs are efficiently incorporated into the foetus head. Indeed the inclusion of arachidonic acid in the supplement increases its concentration, although not up to the RD- level. This emphasises the need to balance the supplementation. The appropriate balance can then be assessed experimentally.

## Claims

1. An edible formulation comprising arachidonic acid (ARA) in an amount adapted to deliver a dosage of from 150mg to 1 g/day ARA.

2. A formulation according to claim 1 which is adapted to deliver from 250 to 500 mg/day ARA.

3. A formulation according to claim 1 to 2 which is additionally adapted to deliver docosahexaenoic acid (DHA).

4. A formulation according to any preceding claim which is adapted to deliver a dosage of from 400 to 600 mg/day DHA.

5. A formulation according to any preceding claim wherein the ratio of ARA:DHA is from 1:5 to 5:1, such as from 1:1 to 1:2.

6. An edible formulation comprising from 150 to 700 mg ARA which is intended to be ingested once per day.

7. An edible formulation comprising from 75 to 350 mg ARA which is adapted to be ingested twice per day.

8. An edible formulation according to any preceding claim which is a food or nutritional supplement.

9. An edible formulation according to any preceding claim which is a pharmaceutical composition.

10. A pharmaceutical composition comprising ARA and DHA at a ratio of ARA:DHA at from 1:1 to 1:2.

11. A foodstuff comprising from 0.1 to 5% ARA.

12. The use of ARA as a dietary or nutritional supplement for a woman who is:
a. pregnant and at an age of from 15 to 20;
b. pregnant and at an age of from 40 to 60, such as from 50 to 55;
c. pregnant with her fourth, fifth or subsequent child;
d. pregnant with twins, triplets or quadruplets;
e. pregnant and is from 1 to 3 months into her pregnancy;
f. pregnant as a result of in vitro fertilisation (IVF) or who is undergoing IVF treatment but not yet pregnant;
g. pregnant at from 20 or more weeks of gestation;
h. pregnant and is malnourished, poorly or marginally nourished, suffering from malnutrition or malabsorption or deficient in one or more essential fatty acids;
i. trying to become pregnant;
j. pregnant, for promoting the intra-uterine growth of health of a foetus; or
k. lactating, for increasing the level of ARA or EPA in the woman's breast milk.

13. The use according to claim 12 wherein the ARA is ingested at from 150 to 700, such as from 250 to 500, mg/day.

14. The use of ARA as a dietary or nutritional supplement for a human who is over 50 years old, preferably over 65 years old.

15. The use of ARA as a dietary or nutritional supplement for a non-human mammal which is pregnant or lactating.

16. The use of ARA for the manufacture of a medicament for assisting in the prophylaxis, prevention, amelioration or treatment of a disease or condition associated with an abnormal or low level of an n-3 or n-6 PUFA in the blood, wherein, for instance, the disease or condition is a neuronal disease, such as schizophrenia, cystic fibrosis, idiopathic immunoglobulin A nephropathy, multiple sclerosis, retinitis pigmentosis, Usher's syndrome, celiac disease, macular degeneration, Parkinsons' disease, osteoporosis, Alzheimer's disease or phenylketonuria.

17. The use of ARA for promoting lactation and/or reproductive efficiency or success, or fertility in a human or non-human female mammal.

18. The use according to any of claims 12 to 17 which additionally includes DHA.

19. The use of ARA and DHA in an edible formulation at an ARA:DHA ratio that increases the ARA level in blood.

20. The use according to claims 12 to 19, wherein the ratio of ARA:DHA is from 1:5 to 5:1, preferably from 1:1 to 1:2.

21. The use according to any of claims 19 to 20 for a person who is a diabetic, an alcoholic, a drug abuser, smoker or who is immunocompromised or has an abnormal immune level.
